(19) Europäisches Patentamt
European Patent Office
Office européen des brevets

(11) Veröffentlichungsnummer: **0 013 385**
**B1**

(12) **EUROPÄISCHE PATENTSCHRIFT**

(45) Veröffentlichungstag der Patentschrift: **27.01.82**

(21) Anmeldenummer: **79105247.5**

(22) Anmeldetag: **18.12.79**

(51) Int. Cl.³: **C 07 C 45/72,** C 07 C 45/73, C 07 C 45/74, C 07 C 49/04, C 07 C 47/02 // B01J23/56, B01J37/00

(54) **Verfahren zur Herstellung von aliphatischen Carbonylverbindungen.**

(30) Priorität: **10.01.79 DE 2900692**

(43) Veröffentlichungstag der Anmeldung: **23.07.80 Patentblatt 80/15**

(45) Bekanntmachung des Hinweises auf die Patenterteilung: **27.01.82 Patentblatt 82/4**

(84) Benannte Vertragsstaaten: **BE CH DE FR GB IT NL**

(56) Entgegenhaltungen:
**DE-A-1 568 457**
**DE-A-2 615 308**
**DE-A-2 625 540**
**US-A-2 064 254**
**CHEMICAL ABSTRACTS, Band 86, Nr. 1, 3. Januar 1977, Seite 426**

(73) Patentinhaber: **BASF Aktiengesellschaft, Carl-Bosch-Strasse 38, D-6700 Ludwigshafen (DE)**

(72) Erfinder: **Heilen, Gerd, Dr. Chem., Schifferstadter Strasse 1B, D-6720 Speyer (DE)**
Erfinder: **Nissen, Axel, Dr. Chem., Panoramastrasse 51, D-6906 Leimen (DE)**
Erfinder: **Woertz, Otto, Dr. Chem., Bruesseler Ring 51, D-6700 Ludwigshafen (DE)**

EP 0 013 385 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von aliphatischen Carbonylverbindungen

Die vorliegende Erfindung betrifft ein neues Verfahren zur Herstellung von aliphatischen Carbonylverbindungen durch Aldolkondensation von Carbonylverbindungen der allgemeinen Formel I

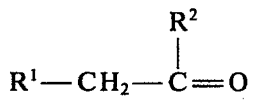
(I)

in der $R^1$ und $R^2$ für Wasserstoff oder eine $C_1$—$C_8$-Alkylgruppe stehen, unter hydrierenden Bedingungen in Gegenwart von Wasserstoff.

Die Aldolselbstkondensation von Aldehyden und Ketonen in Gegenwart von Wasserstoff sowie von Katalysatorsystemen, die sowohl kondensierende als auch hydrierende Eigenschaften haben, ist für zahlreiche spezielle Fälle bekannt.

So erhält man beispielsweise nach der DE-AS 1 643 044 Methylisobutylketon aus Aceton in Gegenwart von Wasserstoff sowie eines stark sauren, zum Teil mit Palladium beladenen Ionenaustauschers. Eine ähnliche Reaktion mit verschiedenen Ketonen läßt sich nach der DE-AS 1 922 755 an einem Kontakt aus Zr-, Hf-, Ti- oder Sn-Phosphat, welcher zusätzlich Palladium enthält, ausführen.

Die hierbei erzielbaren Verfahrensergebnisse befriedigen jedoch nur hinsichtlich der Selektivität in bezug auf die gewünschten Verfahrensprodukte, weit weniger jedoch hinsichtlich des Umsatzes, der sich hier nur auf Kosten der Selektivität erhöhen läßt.

Nach der GB-PS 1 014 273 lassen sich Aldehyde in Gegenwart von Wasserstoff an oxidischen, Palladium enthaltenden Kontakten wie $Al_2O_3$, MgO, ZnO, $Ca(OH)_2$ u. ä., in der Gasphase zu den entsprechenden dimeren gesättigten Aldehyden, z. B. 2-Methylpentanal aus Propionaldehyd, umsetzen, jedoch kommt dieses Verfahren trotz der befriedigenden Selektivität von 73—82% wegen der äußerst geringen Raum-Zeit-Ausbeuten von 24—34 g Produkt pro Stunde und pro Liter Reaktionsraum für technische Zwecke nicht in Betracht.

Auch das Verfahren der US-PS 2 485 989 zur Herstellung von 2-Äthylhexanal aus n-Butyraldehyd in Gegenwart von Wasserstoff, eines Pd/Kohle-Katalysators und Kaliumhydroxid empfiehlt sich aus technisch-wirtschaftlichen Gründen nicht, weil die Selektivitäten mit 64% nur mäßig sind und weil nach beendeter Reaktion eine Neutralisation und somit ein zusätzlicher Verfahrensschritt unter zusätzlichem Materialverbrauch erforderlich ist.

Die genannten Verfahren haben insgesamt den Nachteil, daß sie sich, wenn überhaupt, nur für spezielle Fälle eignen.

Aus der DE-OS 2 615 308 ist es ferner bekannt, höhere Ketone des Typs

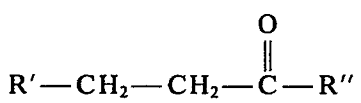

wobei R′ und R″ Alkylgruppen bedeuten, durch gemischte Aldolkondensation eines Aldehyds

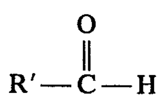

mit einem Methylalkylketon

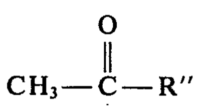

herzustellen, indem man die beiden Komponenten bei höheren Temperaturen in Gegenwart von Wasserstoff und eines Katalysatorsystems umsetzt, welches sowohl kondensierende als auch hydrierende Eigenschaften hat und als aktive Bestandteile ein Metall der VIII. Gruppe des Periodensystems sowie ein Oxid eines Seltenen Erdmetalls enthält.

Wie aus dem experimentellen Teil dieser DE-OS hervorgeht, findet hierbei praktisch weder eine Selbstkondensation des Aldehyds noch eine Selbstkondensation des Ketons statt. Hieraus war zu schließen, daß sich dieses Verfahren, zumindest für technische Zwecke, lediglich für die gemischte Aldolkondensation eines Aldehydes mit einem Keton eignet.

Der Erfindung lag die Aufgabe zugrunde, sowohl Aldehyde als auch Ketone unter gleichartigen Bedingungen mit hoher Wirtschaftlichkeit der Aldolkondensation unter hydrierenden Bedingungen zu unterwerfen. Diese Aufgabe hat besonders wirtschaftliche Bedeutung für den Fall, daß die

Verfahrensprodukte nicht in allzu großen Mengen benötigt werden. Sind die Verfahrensprodukte, die Verfahrensweisen und die Katalysatoren jeweils unterschiedlich, so sind verschiedene Anlagen erforderlich, die bei begrenztem Bedarf jedoch weitgehend ungenutzt bleiben. Legt man die Anlagen entsprechend klein aus, so sind sie bei Spitzenbedarf unflexibel und erfordern überdies fast den gleichen Arbeitsaufwand wie eine Großanlage. Ein auf die definitionsgemäßen Ausgangsverbindungen universell anwendbares Verfahren würde den genannten Nachteil naturgemäß beheben.

Es wurde gefunden, daß man aliphatische Carbonylverbindungen durch Aldolselbstkondensation von Carbonylverbindungen der allgemeinen Formel I

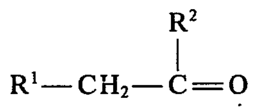

(I)

in der $R^1$ und $R^2$ für Wasserstoff oder eine $C_1—C_8$-Alkylgruppe stehen, in Gegenwart von Wasserstoff und eines Katalysators, der sowohl kondensierende als auch hydrierende Eigenschaften hat, auf wirtschaftliche Weise erhält, wenn man hierzu ein Katalysatorsystem verwendet, dessen aktive Bestandteile aus 1—90 Gewichtsprozent eines Edelmetalles der VIII. Gruppe des Periodensystems und 10—99 Gewichtsprozent eines Oxids oder Salzes eines Seltenen Erdmetalls oder einer Mischung verschiedener derartiger Oxide und/oder Salze bestehen.

Derartige Katalysatorsysteme, die aus der eingangs erwähnten DE-OS 2 615 308 bekannt sind, enthalten als Edelmetall der VIII. Gruppe des Periodensystems vornehmlich Palladium oder daneben auch Platin. Die übrigen Edelmetalle — Ruthenium, Rhodium, Iridium und Osmium — sind meistens ebenfalls geeignet, kommen jedoch aus wirtschaftlichen Gründen in der Regel weniger in Betracht.

Als Verbindungen der Seltenen Erdmetalle (im folgenden als SE-Verbindungen bezeichnet) eignen sich vornehmlich die Oxide, und zwar besonders das Lanthanoxid ($La_2O_3$), das Samariumoxid ($Sm_2O_3$), das Gadoliniumoxid ($Gd_2O_3$) und das Holmiumoxid ($Ho_2O_3$) sowie vor allem das Ceroxid ($Ce_2O_3$), das Praseodymoxid ($Pr_2O_3$) und das Neodymoxid ($Nd_2O_3$). Verwendet man das handelsübliche $CeO_2$, so wird dieses unter den Reaktionsbedingungen zum $Ce_2O_3$ reduziert.

Anstelle der Oxide lassen sich erfindungsgemäß auch die Salze der Seltenen Erdmetalle verwenden, beispielsweise die Nitrate, Sulfate, Phosphate, Chloride und Carbonate. Bevorzugt werden indes die Salze organischer Säuren wie die Acetate, Propionate, Phenolate, Benzolsulfonate, Toluolsulfonate und besonders die organischen Salze der höheren Fettsäuren, z. B. der Stearinsäure. Sind die Salze in der Ausgangsverbindung löslich, so können sie dieser auch vor der Reaktion beigemischt werden, so daß diese Mischung nur noch über einen herkömmlichen Hydrierkontakt aus Edelmetallen der VIII. Gruppe geleitet zu werden braucht. Diese Verfahrensweise empfiehlt sich besonders in den Fällen, in denen man die Umsetzung an einem bereits bestehenden herkömmlichen definitionsgemäßen Hydrierkontakt ohne den Aufwand eines Katalysatorwechsels auf die erfindungsgemäße Reaktion umstellen möchte. Das Verfahrensprodukt braucht dann von dem SE-Salz lediglich abdestilliert zu werden.

Im übrigen ist es nicht erforderlich, die reinen SE-Verbindungen zu verwenden, vielmehr eignen sich ebensogut deren Gemische, z. B. die handelsüblichen SE-Oxide und -Salze technischer Reinheit mit etwa 90gew.-%igem Anteil eines SE-Oxids bzw. -Salzes und dem Rest aus mehreren begleitenden anderen SE-Verbindungen.

Der Begriff Katalysatorsystem soll verdeutlichen, daß es lediglich auf die gleichzeitige Gegenwart der beiden Komponenten, Edelmetall der VIII. Gruppe und SE-Verbindung, während der Umsetzung ankommt. Demgemäß stellt sich die erfindungsgemäß erwünschte Wirkung bereits dann ein, wenn das Edelmetall und die SE-Verbindung gemeinsam in einer Suspension der eingesetzten Carbonylverbindung I oder einer organischen Lösung von I vorliegen. Ebenso verhält es sich, wenn es sich um eine Suspension eines Trägerkatalysators mit einem der definitionsgemäßen Edelmetalle — z. B. Pd auf Aktivkohle — und eines SE-Oxid-Trägerkatalysators — z. B. mit Aluminiumoxid als Träger — handelt.

Derartige Verfahrensweisen sind prinzipiell möglich und häufig auch für Umsetzungen im kleineren Maßstab oder im halbtechnischen Maßstab geeignet. Für den kontinuierlichen technischen Betrieb empfiehlt es sich jedoch aus allgemein bekannten verfahrenstechnischen Gründen, den Katalysator in einer Reaktorsäule fest anzuordnen und Wasserstoff und Carbonylverbindung I bzw. eine Lösung von I über ein derartiges Festbett zu leiten.

Für diesen Zweck verwendet man vorzugsweise Trägerkatalysatoren, auf welche das Edelmetall, z. B. das Palladium, und die SE-Verbindung gemeinsam aufgebracht sind. Man kann solche Trägerkatalysatoren herstellen, indem man das Trägermaterial mit einer wäßrigen Lösung, die ein Pd-Salz wie Palladiumnitrat sowie ein SE-Salz im entsprechenden Mengenverhältnis enthält, imprägniert, trocknet und im Luftstrom erhitzt, wobei sich das SE-Oxid bildet. Das Pd-Metall bildet sich dann unter den hydrierenden Bedingungen von selber, jedoch kann man auch den Trägerkatalysator zu diesem Zwecke einer gesonderten Hydrierung unterwerfen. Für die übrigen Edelmetalle der VIII. Gruppe gilt das gleiche.

Als Trägermaterialien eignen sich z. B. Aktivkohle, Aluminiumoxid und Kieselgel in Form von

Tabletten, Granalien, Kugeln und Strängen mit Durchmessern von 2—20 mm und einer Längenausdehnung von 2—50 mm.

Eine Schüttung von 1 Liter derartiger Trägerkatalysatoren enthält je nach Raumform und Gesamtoberfläche des Trägers etwa 10—150 g aktiver Katalysatorbestandteile.

Allgemein beträgt das Gewichtsverhältnis vom Edelmetall der VIII. Gruppe zur SE-Verbindung 1 : 99—90 : 10, jedoch sind in aller Regel solche Katalysatoren zu bevorzugen, in denen dieses Verhältnis zwischen 5 : 95 und 80 : 20 liegt. Bevorzugt werden Trägerkatalysatoren, die 2—5 Gew.-% eines SE-Oxids und 0,2—0,5 Gew.-% Palladium enthalten, bezogen auf die Gesamtmenge des Katalysators.

Um 1 Mol der Carbonylverbindung I in flüssiger Phase — der bevorzugten Ausführungsform des Verfahrens — bei 180° C und einem Wasserstoffdruck von 30 bar quantitativ oder praktisch quantitativ umzusetzen, benötigt man je nach Katalysatorform etwa 2 bis 10 ml der vorstehend beschriebenen Trägerkatalysatoren, wobei die Reaktionszeit etwa 1 bis 10 Stunden beträgt. Hierbei handelt es sich um Richtwerte, die sich nach bekannten Gesetzmäßigkeiten mit den Reaktionsbedingungen ändern, wie jeweils durch einige Versuche unschwer zu ermitteln ist. Von der Art der Carbonylverbindung I sind die Reaktionsbedingungen weithin unabhängig, so daß es das erfindungsgemäße Verfahren gestattet, in ein und derselben Anlage ohne Katalysatorwechsel verschiedenartige Carbonylverbindungen I umzusetzen.

Das Verfahren gelingt mit gutem Erfolg bereits bei Normaldruck, jedoch kann zur Erhöhung der Reaktionsgeschwindigkeit auch ein Druck bis zu 100 bar angewendet werden. Noch höhere Drucke bringen im Verhältnis zum hierfür erforderlichen technischen Aufwand normalerweise keine Vorteile mehr. Im allgemeinen erzielt man die wirtschaftlich günstigsten Ergebnisse bei Drucken im Bereich von 1—50 bar.

Für die Reaktionstemperatur empfiehlt sich ein Bereich von 20—250, vorzugsweise 20—220° C. Bei tieferen Temperaturen als 20° C sinkt die Reaktionsgeschwindigkeit merklich ab, und höheren Temperaturen als 250° C werden durch Nebenreaktionen, insbesondere fortschreitende Aldolkondensationen, zunehmend Grenzen gesetzt. Bemerkenswerterweise findet indes auch mit höheren Temperaturen nur eine geringfügige Zunahme der Hydrierung der eingesetzten und entstehenden Carbonylverbindungen zu den entsprechenden Alkoholen statt, sofern die Hydrierung der intermediär entstehenden olefinischen Doppelbindung noch nicht beendet ist. Die selektive Wirkung der erfindungsgemäßen Katalysatoren ist somit, was als besonderer Vorteil zu werten ist, weitgehend temperaturunabhängig.

Sofern die Carbonylverbindungen I unter den Reaktionsbedingungen flüssig sind, erübrigt sich die Mitverwendung eines Lösungsmittels, jedoch kann die Gegenwart eines Lösungsmittels auch in diesen Fällen insofern ein Vorteil sein, als dadurch Nebenreaktionen, wie fortschreitenden Aldolkondensationen, entgegengewirkt wird. Sind die Aldehyde fest, so sind sie in Lösung zu bringen.

Als Lösungsmittel eignen sich alle unter den Reaktionsbedingungen inerten Flüssigkeiten, beispielsweise $C_5$—$C_8$-Paraffine, Cyclohexan, Methanol, Äthanol, Isopropanol, Äthylacetat sowie Toluol oder Xylol.

Die Menge des Lösungsmittels ist nicht kritisch und beträgt üblicherweise das 0,5—10fache der Menge des Aldehyds.

Die erfindungsgemäße Reaktion läßt sich wie folgt wiedergeben:

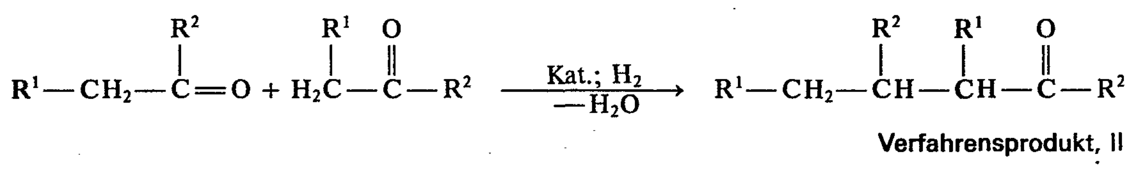


Verfahrensprodukt, II

Sofern $R^2$ ein Rest des Typs $R^3—CH_2—$ ist und $R^3 \neq R^1$ ist, erhält man nach Maßgabe der Reaktionsfähigkeit der beiden $\alpha$-$CH_2$-Gruppen ein Bemisch zweier isomerer Carbonylverbindungen als Verfahrensprodukte II.

Technisch besonders wichtige Ausgangsverbindungen I sind Aldehyde ($R^2$=H), in denen $R^1$ Wasserstoff oder ein verzweigter oder vorzugsweise linearer Alkylrest mit 1—4 C-Atomen ist. So erhält man aus

Acetaldehyd → n-Butyraldehyd
Propionaldehyd → 2-Methylpentanal
n-Butyraldehyd → 2-Äthylhexanal
n-Pentanal → 2-Propylheptanal.

Bevorzugte Ketone sind im Hinblick auf die Verfahrensprodukte Methylketone ($R^1$=H), in denen $R^2$ eine Alkylgruppe mit 1—3 C-Atomen ist. Da die $\alpha$-Methylgruppe reaktionsfähiger ist als eine $\alpha$-Methylengruppe, entstehen im Falle gemischter Ketone I weitgehend einheitliche Ketone II als Verfahrensprodukte. Beispielsweise erhält man aus

Aceton → 4-Methylpentan-2-on
Methyläthylketon → 5-Methylheptan-3-on
Diäthylketon → 4-Methyl-5-äthylheptan-3-on
Methylpropylketon → 6-Methylnonan-4-on

Das erfindungsgemäße Verfahren kann im übrigen nach den üblichen Techniken vorgenommen werden. Das gleiche gilt für die Aufarbeitung der Reaktionsgemische auf die Verfahrensprodukte.

Die Verfahrensprodukte sind bekanntermaßen teils selber wichtige Endprodukte und teils wichtige Zwischenprodukte für die Herstellung von z. B. Pflanzenschutz- und Arzneimitteln.

## Beispiel 1

### Herstellung von 4-Methylpentan-2-on

Ein Rohrreaktor von 3 l Inhalt und 4,5 cm innerem Durchmesser wurde mit 1800 g 5—10 mm langen und 4 mm dicken Strängen eines Trägerkatalysators aus $\gamma$-$Al_2O_3$ in unregelmäßiger Schüttung gefüllt. Der Katalysator, der als aktive Masse 5 Gewichtsprozent $Pr_2O_3$ und 0,5 Gewichtsprozent Pd enthielt wurde in üblicher Weise durch Imprägnieren der $\gamma$-$Al_2O_3$-Stränge mit einer wäßrigen Lösung der Katalysatorkomponenten und anschließendes Trocknen und Erhitzen der imprägnierten Stränge im Luftstrom hergestellt. Zur Bereitung der Lösung wurde eine Suspension aus 75 ml Wasser und 100 g Pr-Oxid mit 180 g 65%iger Salpetersäure und anschließend mit 85 g einer 11%igen wäßrigen Pd-Nitratlösung versetzt. Die hieraus resultierende Lösung wurde sodann mit Wasser auf ein Volumen von 1840 ml aufgefüllt. Als Pr-Oxid wurde das handelsübliche Mischoxid der Bruttoformel $Pr_6O_{11}$ eingesetzt.

Durch diesen Rohrreaktor, der unter einem Wasserstoffdruck von 25 bar stand, wurden bei 150° C in der sogenannten Rieselfahrweise, d. h. von oben, stündlich 1,5 l Aceton eingeleitet. Hieraus ergibt sich eine mittlere Verweilzeit von rund 2 Stunden, innerhalb derer ein Acetonumsatz von 38% erzielt wurde. Die Ausbeute an 4-Methylpentan-2-on, bezogen auf den Umsatz, betrug 91%; daneben fielen noch 1% Isopropanol, 3% des doppelseitigen Aldolisierungsproduktes 2,6-Dimethylheptan-4-on und 2% 4-Methylpent-3-en-2-on an. Da die letztgenannte Verbindung eine Vorstufe des 4-Methylpentan-2-ons ist und zur Hydrierung in die Synthese zurückgeführt werden kann, betrug die Ausbeute an den gewünschten Wertprodukten insgesamt 92%. Die Bestimmung von Umsatz und Ausbeuten wurde gaschromatographisch vorgenommen.

## Beispiel 2

### Herstellung von 2-Methylpentanal

In der Apparatur von Beispiel 1 wurde Propionaldehyd an 2100 g eines Trägerkontaktes umgesetzt, der 0,5 Gewichtsprozent Pd und 5 Gewichtsprozent $Pr_2O_3$ auf $SiO_2$ als Trägermaterial enthielt. Der Katalysator hatte die gleiche Raumform wie der von Beispiel 1 und wurde auf analoge Weise hergestellt.

Bei einem stündlichen Zulauf von 2,5 l Propionaldehyd, der einer mittleren Verweilzeit von rund 1,2 Stunden entspricht, einer Temperatur von 130° C sowie einem Wasserstoffdruck von 25 bar wurde ein Propionaldehyd-Umsatz von 38% erzielt. Die hierauf bezogene Ausbeute an 2-Methylpentanal betrug 83%. Daneben fielen noch 3% Propanol, 5% eines Gemisches aus 2,4-Dimethylhepta-2,4-dienal und 2,4-Dimethylhept-2-enal, 0,5% 2-Methylpentanol und 4% 2-Methylpent-2-enal an. Da die letztgenannte Verbindung nach Rückführung in die Synthese ebenfalls in das 2-Methylpentanal überführt werden kann, betrug die Ausbeute an Wertprodukten somit insgesamt 87%.

Die Bestimmung von Umsatz und Ausbeuten wurde gaschromatographisch vorgenommen.

## Beispiel 3

### Herstellung von 2-Methylpentanal

In der Apparatur von Beispiel 1 wurde Propionaldehyd an 1800 g eines Trägerkatalysators umgesetzt, der 0,5 Gewichtsprozent Pd und 5 Gewichtsprozent $CeO_2$ auf $\gamma$-$Al_2O_3$ als Trägermaterial enthielt. Der Katalysator hatte die gleiche Raumform wie der von Beispiel 1 und wurde auf analoge Weise hergestellt.

Bei einem stündlichen Zulauf von 3 l Propionaldehyd, der einer mittleren Verweilzeit von rund einer Stunde entspricht, einer Temperatur von 170° C und einem Wasserstoffdruck von 35 bar wurde ein Umsatz von 33% erzielt. Die hierauf bezogene Ausbeute an 2-Methylpentanal betrug 84%. Daneben

wurden noch 2% Propanol, 6% des in Beispiel 2 genannten Dimethylheptenal-Gemisches, 2% 2-Methylpentanol sowie 3% 2-Methylpent-2-enal erhalten. Die letztgenannte Verbindung ist, wie im Beispiel 2, den Wertprodukten zuzurechnen, so daß deren Ausbeute insgesamt 87% betrug.

Bei Erhöhung des Durchsatzes auf 3,5 l/h, der Temperatur auf 200°C und des Wasserstoffdruckes auf 50 bar wurde ein Umsatz von 82% erzielt. Dafür sank die Ausbeute auf 82% (davon 79% 2-Methylpentanal) und es wurden 10% des Dimethylheptenal-Gemisches als Nebenprodukt erhalten. Die Menge der übrigen Nebenprodukte blieb praktisch unverändert. Die Analysenwerte wurden gaschromatographisch bestimmt.

### Beispiel 4

### Herstellung von 2-Methylpentanal

In einem diskontinuierlichen Versuch wurden 150 g Propionaldehyd bei 180°C, einem Wasserstoffdruck von 25 bar und einer Reaktionszeit von 2 h an einem Trägerkatalysator umgesetzt, der 0,5 Gewichtsprozent Pd und 5 Gewichtsprozent $Pr_2O_3$ auf $\gamma$-$Al_2O_3$-Pulver als Trägermaterial enthielt. Der Katalysator wurde auf analoge Weise hergestellt wie in Beispiel 1.

Der Umsatz an Propionaldehyd betrug 95% und die hierauf bezogene Ausbeute an 2-Methylpentanal und -pentenal 88%. Diese Werte wurden gaschromatographisch bestimmt.

### Beispiel 5

### Herstellung von 2-Methylpentanal

In einem diskontinuierlichen Versuch wurden 150 g Propionaldehyd bei 180°C, einem Wasserstoffdruck von 25 bar und einer Reaktionszeit von 4 h an einem Trägerkatalysator umgesetzt, der 0,2 Gewichtsprozent Pd und 2 Gewichtsprozent $La_2O_3$ auf $\gamma$-$Al_2O_3$-Pulver als Trägermaterial enthielt. Der Katalysator wurde auf analoge Weise hergestellt wie in Beispiel 1. Der Umsatz an Propionaldehyd betrug 91% und die hierauf bezogenen Ausbeuten an 2-Methylpentanal und -pentenal 86%.

Diese Werte wurden gaschromatographisch bestimmt.

### Beispiel 6

### Herstellung von 2-Methylpentanal

In einem diskontinuierlichen Versuch wurden 150 g Propionaldehyd bei 180°C, einem Wasserstoffdruck von 25 bar und einer Reaktionszeit von 4 h mit 2 g eines Pd/Aktivekohle Katalysators, der 10 Gewichtsprozent Pd enthielt, und 2 g Neodymstearat umgesetzt.

Der Umsatz an Propionaldehyd betrug 93% und die hierauf bezogene Ausbeute an 2-Methylpentanal und -pentenal 84%. Diese Werte wurden gaschromatographisch bestimmt.

### Beispiel 7

### Herstellung von 2-Äthylhexanal

Unter den Bedingungen von Beispiel 4, jedoch mit einer Reaktionszeit von 3 h wurde n-Butyraldehyd zu 90% umgesetzt. Die hierauf bezogene Ausbeute an 2-Äthylhexanal betrug 87%. Diese Werte wurden gaschromatographisch bestimmt.

### Beispiel 8

### Herstellung von 2-(n-Propyl)-heptanal

Unter den Bedingungen von Beispiel 4 wurde n-Pentanal zu 82% umgesetzt. Die hierauf bezogenen Ausbeuten an 2-(n-Propyl)-heptanal und -hept-2-enal betrug 82%. Diese Werte wurden gaschromatographisch bestimmt.

**Patentanspruch**

Verfahren zur Herstellung von aliphatischen Carbonylverbindungen durch Aldolselbstkondensation von Carbonylverbindungen der allgemeinen Formel I

$$R^1-CH_2-\overset{\overset{R^2}{|}}{C}=O \qquad \text{(I)}$$

in der $R^1$ und $R^2$ für Wasserstoff oder eine $C_1-C_8$-Alkylgruppe stehen, in Gegenwart von Wasserstoff und eines Katalysators, der sowohl kondensierende als auch hydrierende Eigenschaften hat, dadurch gekennzeichnet, daß man hierzu ein Katalysatorsystem verwendet, dessen aktive Bestandteile aus 1—90 Gewichtsprozent eines Edelmetalles der VIII. Gruppe des Periodensystems und 10—99 Gewichtsprozent eines Oxids oder Salzes eines Seltenen Erdmetalls oder einer Mischung verschiedener derartiger Oxide und/oder Salze bestehen.

**Claim**

A process for the preparation of an aliphatic carbonyl compound by aldol autocondensation of a carbonyl compound of the general formula I

$$R^1-CH_2-\overset{\overset{R^2}{|}}{C}=O \qquad \text{(I)}$$

where $R^1$ and $R^2$ are hydrogen or $C_1-C_8$-alkyl, in the presence of hydrogen and of a catalyst which possesses both condensing and hydrogenating properties, characterized in that a catalyst system is used whose active components are from 1 to 90 per cent by weight of a noble metal of group VIII of the periodic table and from 10 to 99 per cent by weight of an oxide or salt of a rare earth metal, or of a mixture of different oxides and/or salts of rare earth metals.

**Revendication**

Procédé pour la préparation de composés carbonylés aliphatiques par condensation avec eux-mêmes ou aldolisation de composés carbonylés de formule générale I

$$R^1-CH_2-\overset{\overset{R^2}{|}}{C}=O \qquad \text{(I)}$$

dans laquelle chacun des symboles $R^1$ et $R^2$ est mis pour un hydrogène ou un groupe alcoyle à 1—8 C, en présence d'hydrogène et d'un catalyseur qui présente des propriétés à la fois de condensation et d'hydrogénation, caractérisé en ce qu'on utilise à cette fin un système catalyseur dont les composants actifs sont constitués par 1 à 90% en poids d'un métal noble du groupe VIII du système périodique et par 10 à 99% en poids d'un oxyde ou sel d'un métal du groupe des terres rares ou d'un mélange de différents oxydes et/ou sels de ce genre.